# EUROPÄISCHE PATENTSCHRIFT

(11) **EP 3 549 684 B1**
(45) Veröffentlichungstag und Bekanntmachung des Hinweises auf die Patenterteilung: **21.09.2022**
(21) Anmeldenummer: 18165935.0
(22) Anmeldetag: 05.04.2018
(51) Int. Cl.: B08B 3/02, B08B 5/04, A47L 11/30, A47L 11/40

(54) **PORTABLE REINIGUNGSVORRICHTUNG ZUR FLÜSSIGREINIGUNG SENSIBLER BAUTEILE**
PORTABLE CLEANING DEVICE FOR LIQUID CLEANING OF SENSITIVE COMPONENTS
DISPOSITIF DE NETTOYAGE PORTABLE DESTINÉ AU NETTOYAGE LIQUIDE DES COMPOSANTS SENSIBLES

(43) Veröffentlichungstag der Anmeldung: 09.10.2019
(60) Teilanmeldung: 20172143.8
(73) Patentinhaber: Airbus Defence and Space GmbH, 82024 Taufkirchen (DE)
(72) Erfinder: Bose, Guido, 88090 Immenstaad (DE)
(74) Vertreter: Daub, Thomas

(56) Entgegenhaltungen:
- EP-A1- 2 546 002
- WO-A1-2014/142877
- WO-A2-2012/044114
- DE-A1-102013 206 875
- FR-A1- 2 756 503
- US-A- 4 597 124
- US-A1- 2013 276 831

## Beschreibung

### Stand der Technik

Die Erfindung betrifft eine portable Reinigungsvorrichtung zur Flüssigreinigung sensibler Bauteile, insbesondere in der Luft- und Raumfahrt.

Es ist bereits eine portable Reinigungsvorrichtung mit zumindest einem Reinigungskopf, welche zumindest eine Aufbringeinheit zu einem Aufbringen eines Reinigungsfluids und zumindest eine Absaugeinheit zu einem Absaugen des Reinigungsfluids aufweist, vorgeschlagen worden.

Ferner werden verunreinigte sensible Bauteile, insbesondere Luft- und/oder Raumfahrtbauteile, bisher mit Reinigungstüchern gereinigt; hier werden die Bauteile zumeist zusätzlich kontaminiert durch die Fasern des Tuches und die Verunreinigung wird durch die mechanische Bewegung in das Bauteil eingebracht. Zudem ist der Reinigungsvorgang nicht kontaktlos, daher besteht die Möglichkeit, dass die Bauteile während des Reinigungsprozesses zusätzlich verunreinigt werden. Eine Bioburden-Evakuierung (Reinigung des Bauteils von biologischem Material wie z.B. Mikroben und Sporen) zur Vorbereitung von Bauteilen für Planetenmissionen ist bisher sehr aufwendig. Die Aufgabe der Erfindung besteht insbesondere darin, eine vorteilhaft kostengünstige gattungsgemäße Reinigungsvorrichtung mit verbesserten Eigenschaften hinsichtlich einer Reinigungseffizienz bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

Die Druckschrift US4597124-A offenbart eine Portable Reinigungsvorrichtung gemäß dem Oberbegriff des Anspruchs 1.

### Vorteile der Erfindung

Die Erfindung geht aus von einer portablen Reinigungsvorrichtung zur Flüssigreinigung sensibler Bauteile, gemäß Anspruch 1.

Es wird vorgeschlagen, dass der Reinigungskopf zumindest eine Aufbringeinheit zum Aufbringen eines Trocknungsfluids aufweist, insbesondere eines Trocknungsgases, zu einer Trocknung des sensiblen Bauteils. Vorzugsweise ist die Aufbringeinheit zum Aufbringen eines Trocknungsfluids von der Aufbringeinheit zu einem Aufbringen des Reinigungsfluids verschieden; es wäre jedoch auch denkbar, dass eine Aufbringeinheit zu einem Aufbringen eines Trocknungsfluids und zu einem Aufbringen eines Reinigungsfluids vorgesehen ist. Insbesondere wäre denkbar, dass die Aufbringeinheiten von einer einzigen Aufbringeinheit gebildet sind. Bevorzugt ist die Reinigungsvorrichtung zu einer Reinigung der sensiblen Bauteile zumindest teilweise mittels einer Flüssigkeit vorgesehen. Besonders bevorzugt erfolgt eine Reinigung mittels der Reinigungsvorrichtung kontaktlos, wobei der Reinigungskopf während einer Reinigung insbesondere frei von einer Berührung mit dem zu reinigenden Bauteil ist. Insbesondere ist die Reinigungsvorrichtung dazu vorgesehen, sensible Bauteile kontaktlos mit definierten Flüssigkeiten zu reinigen und gemäß Bioburden-Vorgaben vorzubereiten. Unter einer "portablen Reinigungsvorrichtung" soll hier insbesondere eine Reinigungsvorrichtung zu einer Reinigung eines Bauteils verstanden werden, wobei eine Reinigung handgehalten und/oder freistehend erfolgen kann. Vorzugsweise erfolgt eine Reinigung mittels der Reinigungsvorrichtung zumindest teilweise manuell, wie insbesondere durch eine manuelle Führung des Reinigungskopfs. Bevorzugt soll unter einer "portablen Reinigungsvorrichtung" insbesondere eine Reinigungsvorrichtung verstanden werden, die von einem Bediener transportmaschinenlos transportiert werden kann. Die portable Reinigungsvorrichtung weist insbesondere eine Masse auf, die kleiner ist als 60 kg, bevorzugt kleiner ist als 40 kg, bevorzugt kleiner ist als 20 kg und besonders bevorzugt kleiner ist als 10 kg. Ferner sind verschiedene, einem Fachmann als sinnvoll erscheinende sensible Bauteile denkbar, wie beispielsweise Solarzellen, Leiterplatten, Sensoren, aber auch empfindliche Gehäuse und/oder Verkleidungen oder dergleichen, welche zu einer Reinigung mittels der Reinigungsvorrichtung vorgesehen sind.

Unter einer "Aufbringeinheit" soll in diesem Zusammenhang insbesondere eine Einheit verstanden werden, die zu einem Aufbringen eines Fluids vorgesehen ist. Vorzugsweise umfasst die Einheit eine Führungsleitung zu einer Führung des Fluids sowie ein Ausbringelement, über welches das Fluid zielgerichtet ausgebracht werden kann. Das Ausbringelement ist insbesondere von einer Düse oder einem Diffusor gebildet. Es sind jedoch auch andere, einem Fachmann als sinnvoll erscheinende Ausbringelemente denkbar. Vorzugsweise kann die Aufbringeinheit zu einem gezielten Ausbringen sowohl einer Flüssigkeit als auch eines Gases vorgesehen sein. Das Reinigungsfluid ist insbesondere von einer Reinigungsflüssigkeit gebildet. Es sind verschiedene, einem Fachmann als sinnvoll erscheinende Reinigungsflüssigkeiten denkbar, wie insbesondere Isopropanol, Azeton, verdünnte Schwefelsäure, reiner Alkohol, etc. Ferner soll unter einer "Absaugeinheit" in diesem Zusammenhang insbesondere eine Einheit verstanden werden, welche zu einem Absaugen des Reinigungsfluids aus einem Arbeitsbereich der Reinigungsvorrichtung vorgesehen ist. Vorzugsweise ist die Einheit dazu vorgesehen, ein Luft-Reinigungsfluid-Gemisch aus einem Arbeitsbereich der Reinigungsvorrichtung abzusaugen, wobei anschließend insbesondere eine Trennung des Reinigungsfluids von der Luft erfolgt. Bevorzugt wird mit dem Reinigungsfluid auch in dem Reinigungsfluid gebundener Schmutz abgesaugt. Das Reinigungsfluid wird während eines Betriebs insbesondere zumindest zu einem Großteil von dem sensiblen Bauteil abgesaugt. Vorzugsweise erfolgt eine Absaugung mittels Unterdruck. Besonders bevorzugt weist die Absaugeinheit zumindest eine in einem Bereich eines Ausbringelements der zumindest einen Ausbringeinheit angeordnete Absaugöffnung, welche beispielsweise als eine Düse ausgebildet sein kann. Des Weiteren soll in diesem Zusammenhang unter einem "Trocknungsfluid" insbesondere ein Fluid verstanden werden, welches dazu vorgesehen ist, eine Trocknung des sensiblen Bauteils, insbesondere eine Trocknung des Reinigungsfluids auf dem sensiblen Bauteil, zu beschleunigen. Eine Trocknung wird dabei insbesondere gegenüber einer reinen Lufttrocknung wesentlich beschleunigt. Vorzugsweise wird eine Trocknungszeit mittels des Trocknungsfluids gegenüber einer reinen Lufttrocknung um zumindest 10 %, vorzugsweise um zumindest 30 %, bevorzugt um zumindest 50 % und besonders bevorzugt um zumindest 70 % reduziert. Es sind verschiedene, einem Fachmann als sinnvoll erscheinende Trocknungsfluide denkbar. Besonders bevorzugt ist das Trocknungsfluid von einem Trocknungsgas, wie insbesondere von Stickstoff (N₂), gebildet. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung der portablen Reinigungsvorrichtung kann insbesondere eine vorteilhaft effiziente und kostengünstige Reinigung sensibler Bauteile erreicht werden. Es kann insbesondere mit einem vorteilhaft geringen Aufwand eine vorteilhaft effiziente Reinigung erreicht werden. Insbesondere kann ein vorzugsweise kontaktloses Reinigen und Trocknen in einem Arbeitsgang erfolgen. Hierdurch kann ein flexibles Reinigen erfolgen.

Ferner wird vorgeschlagen, das die portable Reinigungsvorrichtung zumindest eine Kopplungseinheit aufweist, die zu einer lösbaren Kopplung der Reinigungsvorrichtung mit einem Anschlussmodul vorgesehen ist, wobei die Kopplungseinheit zumindest Anschlüsse zur Bereitstellung und zur Abfuhr des Reinigungsfluids und des Trocknungsfluids aufweist. Vorzugsweise weist die Kopplungseinheit zumindest einen Anschluss zu einer Bereitstellung des Reinigungsfluids und/oder des Trocknungsfluids und zumindest einen Abschluss zu einer Abfuhr eines abgesaugten Reinigungsfluids auf. Bevorzugt weist die Kopplungseinheit zumindest einen Anschluss zu einer Bereitstellung des Reinigungsfluids und zumindest einen Anschluss zu einer Bereitstellung des Trocknungsfluids auf. Vorzugsweise werden das Reinigungsfluid und das Trocknungsfluid insbesondere von dem Anschlussmodul bereitgestellt. Unter einer "Kopplungseinheit" soll in diesem Zusammenhang insbesondere eine Einheit verstanden werden, welche zu einer Bereitstellung zumindest eines Anschlusses zu einer Kopplung mit dem Anschlussmodul vorgesehen ist. Vorzugsweise ist die Kopplungseinheit fest mit dem Reinigungskopf gekoppelt und lösbar mit dem Anschlussmodul koppelbar. Vorzugsweise ist die Kopplungseinheit über zumindest eine Steck- und/oder Schraubverbindung werkzeuglos mit dem Anschlussmodul koppelbar. Unter einem "Anschlussmodul" soll in diesem Zusammenhang insbesondere ein Modul verstanden werden, welches zu einer Bereitstellung eines Reinigungsfluids und/oder eines Trocknungsfluids vorgesehen ist und welches zu einer Aufnahme und/oder Reinigung eines abgesaugten Reinigungsfluids vorgesehen ist. Vorzugsweise weist das Modul einen ersten Tank zu einer Aufnahme des Reinigungsfluids, zumindest einen zweiten Tank zu einer Aufnahme des Trocknungsfluids und zumindest einen dritten Tank zu einer Aufnahme eines abgesaugten Reinigungsfluids auf. Besonders bevorzugt ist das Anschlussmodul von einem zumindest im Wesentlichen von der Reinigungsvorrichtung getrennten, insbesondere freistehenden, Modul gebildet. Dadurch kann insbesondere eine vorteilhaft flexible und insbesondere leichte Reinigungsvorrichtung bereitgestellt werden. Es kann insbesondere eine Auslagerung eines Tanks für das Reinigungsfluid sowie eines Tanks für das Trocknungsfluid erreicht werden, wodurch Gewicht eingespart werden kann. Hierdurch kann eine vorteilhaft portable Reinigungsvorrichtung bereitgestellt werden.

Des Weiteren wird vorgeschlagen, dass die portable Reinigungsvorrichtung einen Griffteil aufweist, an welchem der Reinigungskopf angeordnet ist und welcher zu einer manuellen Führung des Reinigungskopfs vorgesehen ist. Vorzugsweise ist der Griffteil von einem Pistolengriff gebildet. Es wäre jedoch auch eine andere, einem Fachmann als sinnvoll erscheinende Griffausgestaltung denkbar. Bevorzugt ist der Griffteil von einem Gehäuse der Reinigungsvorrichtung ausgebildet. Besonders bevorzugt ist der Griffteil zu einer manuellen Führung des Reinigungskopfes während eines Betriebs vorgesehen. Insbesondere wird mittels des Griffteils die gesamte portable Reinigungsvorrichtung bewegt. Unter einem "Griffteil" soll insbesondere ein Teil der Reinigungsvorrichtung verstanden werden, der in einem Betrieb der Reinigungsvorrichtung dazu vorgesehen ist, von einer Hand eines Bedieners umgriffen zu werden. Vorzugsweise bildet der Griffteil eine Grifffläche aus, welche zu einer direkten Kontaktierung durch die Hand des Bedieners vorgesehen ist. Die Grifffläche kann zu einer Bereitstellung eines Komforts beispielsweise gummiert oder beschichtet ausgeführt sein. Durch den Griffteil kann insbesondere eine vorteilhaft einfache und komfortable Führung der portablen Reinigungsvorrichtung erreicht werden. Es kann insbesondere ein vorteilhaft einfach transportables Handgerät bereitgestellt werden. Vorzugsweise kann die Reinigungsvorrichtung hierdurch auch über große Flächen zu einer Reinigung bewegt werden. Bevorzugt ist das zu reinigende sensible Bauteil dadurch nicht an eine Größe der Reinigungsvorrichtung gebunden.

Es wird ferner vorgeschlagen, dass die portable Reinigungsvorrichtung eine zwischen Griffteil und Reinigungskopf angeordnete Verstelleinheit aufweist, über welche eine räumlichen Ausrichtung des Reinigungskopfs relativ zu dem Griffteil einstellbar ist. Vorzugsweise ist über die Verstelleinheit eine räumliche Ausrichtung des Reinigungskopfs relativ zu dem Griffteil einstellbar und fixierbar. Bevorzugt kann der Reinigungskopf mittels der Verstelleinheit in verschiedenen Richtungen relativ zu dem Griffteil bewegt werden. Unter einer "Verstelleinheit" soll in diesem Zusammenhang insbesondere eine Einheit verstanden werden, welche insbesondere zwischen dem Reinigungskopf und dem Griffteil angeordnet ist und welche zu einer Einstellung einer Ausrichtung des Reinigungskopfs relativ zu dem Griffteil vorgesehen ist. Vorzugsweise umfasst die Verstelleinheit zumindest einen flexiblen, insbesondere elastischen, Teilbereich, welcher zu einer flexiblen Führung von Leitungen von dem Griffteil zu dem Reinigungskopf vorgesehen ist. Es sind verschiedene, einem Fachmann als sinnvoll erscheinende Ausgestaltungen der Verstelleinheit denkbar, insbesondere ist die Verstelleinheit jedoch von einem Metallschlauch gebildet. Dadurch kann insbesondere eine vorteilhaft flexible portable Reinigungsvorrichtung bereitgestellt werden. Insbesondere kann die Reinigungsvorrichtung dadurch an eine Reinigungssituation angepasst werden. Es kann insbesondere eine flexible Ausrichtung des Reinigungskopfs erreicht werden.

Es wird weiter vorgeschlagen, dass die portable Reinigungsvorrichtung eine an dem Griffteil angeordnete Bedieneinheit aufweist, welche zu einer Betätigung der zumindest einen Aufbringeinheit und der Absaugeinheit vorgesehen ist. Vorzugsweise ist die Bedieneinheit dazu vorgesehen, die zumindest eine Aufbringeinheit mit einem Reinigungsfluid und/oder einem Trocknungsfluid zu versorgen und/oder die Absaugeinheit mit einer Absaugung zu koppeln. Bevorzugt weist die Bedieneinheit zumindest ein Bedienelement zu einer Versorgung der Aufbringeinheit mit einem Reinigungsfluid, zumindest ein Bedienelement zu einer Versorgung der Aufbringeinheit mit einem Trocknungsfluid und/oder zumindest ein Bedienelement zu einer Kopplung der Absaugeinheit mit einer Absaugung auf. Es ist insbesondere denkbar, dass die Bedieneinheit ein einziges Betätigungselement für die zumindest eine Aufbringeinheit und für die Absaugeinheit aufweist oder für jede Aufbringeinheit und Absaugeinheit jeweils ein Betätigungselement umfasst. Unter einer "Bedieneinheit" soll hier insbesondere eine Einheit verstanden werden, die zumindest ein Bauteil aufweist, welches direkt von einem Bediener betätigbar ist, und die dazu vorgesehen ist, durch eine Betätigung und/oder durch eine Eingabe von Parametern einen Prozess und/oder einen Zustand einer mit der Bedieneinheit gekoppelten Einheit zu beeinflussen und/oder zu ändern. Vorzugsweise weist die Bedieneinheit zumindest ein Bedienelement auf. Unter einem "Bedienelement" soll insbesondere ein Element verstanden werden, das dazu vorgesehen ist, bei einem Bedienvorgang eine Eingabegröße von einem Bediener aufzunehmen und insbesondere unmittelbar von einem Bediener kontaktiert zu werden, wobei ein Berühren des Bedienelements und/oder eine auf das Bedienelement ausgeübte Betätigungskraft sensiert und/oder mechanisch zur Betätigung einer Einheit weitergeleitet werden/wird. Dadurch kann insbesondere eine vorteilhaft einfache und intuitive Bedienung der portablen Reinigungsvorrichtung bereitgestellt werden.

Zudem wird vorgeschlagen, dass der Reinigungskopf eine flexible, die zumindest eine Aufbringeinheit und die zumindest eine Absaugeinheit umgreifende Schirmeinheit aufweist, welche zu einer zumindest teilweisen Abschirmung eines Reinigungsbereichs vorgesehen ist. Vorzugsweise wird der Reinigungsbereich von der Schirmeinheit definiert. Unter einer "Schirmeinheit" soll in diesem Zusammenhang insbesondere eine Einheit verstanden werden, welche zu einer Abschirmung eines räumlichen Bereichs vorgesehen ist, welcher einen Reinigungsbereich der Reinigungsvorrichtung bildet. Vorzugsweise ist die Schirmeinheit zumindest teilweise glockenförmig ausgebildet, wobei ein von der Schirmeinheit umgriffener und durch die Schirmeinheit zumindest teilweise begrenzter Raum den Reinigungsbereich bildet. Bevorzugt ragen die zumindest eine Aufbringeinheit und die zumindest eine Absaugeinheit durch eine Öffnung in der Schirmeinheit in den Reinigungsbereich. Besonders bevorzugt weist die Schirmeinheit zumindest eine weitere Öffnung auf, welche zu einer Angrenzung an das sensible Bauteil vorgesehen ist. Vorzugsweise ist die Schirmeinheit dazu vorgesehen, einen als Reinigungsbereich ausgebildeten räumlichen Bereich zwischen der zumindest einen Aufbringeinheit und der zumindest einen Absaugeinheit und dem sensiblen Bauteil zu einer Seite hin zu begrenzen. Dadurch kann insbesondere eine vorteilhaft gezielte Reinigungswirkung erzielt werden. Es kann insbesondere eine zu starke Streuung des Reinigungsfluids vermieden werden. Ferner kann eine vorteilhafte Absaugleistung der Absaugeinheit bereitgestellt werden. Zudem kann eine erneute Kontamination des sensiblen Bauteils in einem nassen Zustand vermieden werden.

Ferner wird vorgeschlagen, dass der Reinigungskopf zumindest ein Beleuchtungselement zur optischen Ausleuchtung des Reinigungsbereichs oder ein UV-Beleuchtungselement zur biologischen Dekontamination des Bauteils umfasst. Vorzugsweise ist das Beleuchtungselement in dem Reinigungskopf, insbesondere in einem Gehäuse des Reinigungskopfs, integriert. Das Beleuchtungselement ist insbesondere von einem LED-Leuchtmittel gebildet. Ferner wäre auch denkbar, dass der Reinigungskopf ein Beleuchtungselement aufweist, das zu einer optischen Ausleuchtung und zu einer biologischen Dekontamination des sensiblen Bauteils vorgesehen ist. Vorzugsweise weist das UV-Beleuchtungselement in einem Emissionsspektrum einen Primärpeak zwischen 40 nm und 400 nm, vorzugsweise zwischen 240 nm und 280 nm, auf. Dadurch kann insbesondere eine vorteilhafte Beleuchtung des Reinigungsbereichs bereitgestellt werden und/oder eine Reinigungsleistung verbessert werden. Es kann insbesondere eine gezielte Ausleuchtung des Reinigungsbereichs ermöglicht und gewährleistet werden.

Des Weiteren wird vorgeschlagen, dass die portable Reinigungsvorrichtung ein eine Bewegung des Reinigungskopfs in mindestens zwei Dimensionen ermöglichendes Schienensystem aufweist. Vorzugsweise ermöglicht das Schienensystem zumindest eine Bewegung des Reinigungskopfs in einer horizontalen Ebene. Bevorzugt ist das Schienensystem zu einer Führung des Reinigungskopfs vorgesehen. Unter einem "Schienensystem" soll in diesem Zusammenhang insbesondere ein System zu einer definierten Führung des Reinigungskopfs verstanden werden. Vorzugsweise umfasst das Schienensystem zumindest eine Führungsschiene zu einer definierten Führung des Reinigungskopfs. Das Schienensystem umfasst insbesondere zumindest zwei miteinander gekoppelte Linearführungen, die insbesondere jeweils orthogonal zueinander ausgerichtet sind. Dadurch kann insbesondere eine vorteilhaft geführte Bewegung des Reinigungskopfs ermöglicht werden. Hierdurch kann ein vorteilhaftes Reinigungsergebnis erzielt werden.

Die portable Reinigungsvorrichtung weist zumindest eine, den Reinigungskopf und das zu reinigende Bauteil von mehreren Seiten umschließende Haube auf, welche zumindest teilweise aus einem transparenten Material besteht. Vorzugsweise umschließt die Haube den Reinigungskopf und das zu reinigende Bauteil in zumindest einer Ebene vollständig. Bevorzugt umschließt die Haube den Reinigungskopf und das zu reinigende Bauteil zu zumindest drei, vorzugsweise zu zumindest vier und besonders bevorzugt zu zumindest fünf Seiten hin. Besonders bevorzugt ist die Haube insbesondere von einem wannenartigen Behälter, insbesondere mit einem Deckel, gebildet. Der Ausdruck "transparent" soll hier insbesondere eine Lichtdurchlässigkeit eines Werkstoffs bzw. eines Bauteils definieren, wobei der Werkstoff bzw. das Bauteil insbesondere zumindest mehr als 10 %, bevorzugt mehr als 50 % und besonders bevorzugt mehr als 80 % eines Spektralbereichs eines sichtbaren Lichts durchlässt. Vorzugsweise weist der Spektralbereich des sichtbaren Lichts eine Wellenlänge λ von ungefähr 350 nm bis 800 nm auf. Besonders bevorzugt ist ein Gegenstand, der, zumindest entlang einer Richtung betrachtet, vollständig von der transparenten Haube verdeckt ist, für einen Bediener bei einem Blick entlang einer Richtung, welche die transparente Haube und den Gegenstand schneidet, sichtbar. Es sind verschiedene, einem Fachmann als sinnvoll erscheinende Materialien denkbar. Vorzugsweise besteht die Haube zumindest teilweise aus transparentem Acrylglas. Dadurch kann insbesondere eine vorteilhaft zuverlässige Reinigung ermöglicht werden. Es kann insbesondere ein Umherspritzen des Reinigungsfluids vermieden werden und gleichzeitig eine Sichtbarkeit des zu reinigenden Bauteils gewährleistet werden. Hierdurch kann insbesondere eine vorteilhaft gekapselte Reinigung ermöglicht werden.

Die portable Reinigungsvorrichtung weist zumindest ein auf der Haube bewegliches, handgeführtes magnetisches Führungselement auf, welches zu einer indirekten Bewegung des Reinigungskopfs vorgesehen ist, wobei der Reinigungskopf einen Permanentmagneten aufweist. Vorzugsweise ist das handgeführte magnetische Führungselement zu einer indirekten Bewegung des Reinigungskopfs an dem Schienensystem mittels Magnetkraft vorgesehen. Bevorzugt weist das Führungselement einen Handgriff sowie einen Permanentmagneten auf. Dadurch kann insbesondere eine kontaktlose Führung des Reinigungskopfs ermöglicht werden. Es kann insbesondere eine Führung des innerhalb der Haube angeordneten Reinigungskopfs von außerhalb der Haube ermöglicht werden. Hierdurch kann insbesondere eine vorteilhaft gekapselte Reinigung ermöglicht werden.

Zudem wird vorgeschlagen, dass die portable Reinigungsvorrichtung zumindest eine unterhalb des Reinigungskopfs angeordnete Aufnahmewanne zur Aufnahme von nicht abgesaugtem Reinigungsfluid und/oder Trocknungsfluid aufweist. Vorzugsweise ist die Aufnahmewanne unter dem Reinigungskopf und unter dem zu reinigenden Bauteil angeordnet. Bevorzugt bildet die Aufnahmewanne gemeinsam mit der Haube einen zumindest im Wesentlichen abgeschlossenen Aufnahmebereich für den Reinigungskopf und das zu reinigende Bauteil aus. Besonders bevorzugt bildet die Aufnahmewanne einen Teil der Haube aus. Hierdurch kann insbesondere eine vorteilhaft gekapselte Reinigung ermöglicht werden. Es kann insbesondere das Austreten und Verteilen von Reinigungsfluid vermieden werden.

Ferner wird vorgeschlagen, dass die portable Reinigungsvorrichtung zumindest eine Halteeinheit aufweist, welche zu einer im Wesentlichen freien Aufnahme des zu reinigenden sensiblen Bauteils vorgesehen ist. Vorzugsweise ist die Halteeinheit an der Haube und/oder der Aufnahmewanne der portablen Reinigungsvorrichtung angeordnet. Unter einer "Halteeinheit" soll in diesem Zusammenhang insbesondere eine Einheit verstanden werden, die zu einer positionsfesten Aufnahme und Fixierung des zu reinigenden Bauteils relativ zu der Haube der portablen Reinigungsvorrichtung vorgesehen ist. Vorzugsweise ist die Halteeinheit zu einem Einspannen des zu reinigenden Bauteils vorgesehen. Ein Einspannen kann dabei beispielsweise mittels Klemmen und/oder Zwingen erfolgen. Ferner soll in diesem Zusammenhang unter einer "im Wesentlichen freien Aufnahme" des zu reinigenden Bauteils insbesondere verstanden werden, dass in einem fixierten Zustand des zu reinigenden Bauteils zumindest ein Großteil der Außenfläche des zu reinigenden Bauteils, insbesondere zumindest 50%, vorzugsweise zumindest 70% und besonders bevorzugt zumindest 90% der Außenfläche, frei zugänglich und/oder reinigbar ist/sind. Dadurch kann insbesondere eine vorteilhafte Reinigung des zu reinigenden Bauteils ermöglicht werden. Es kann insbesondere eine vorteilhaft definierte und gezielte Reinigung ermöglicht werden.

Des Weiteren wird vorgeschlagen, dass die Halteeinheit mehrere Freiheitsgrade der Bewegung zur Positionierung des zu reinigenden sensiblen Bauteils aufweist. Vorzugsweise weist die Halteeinheit mehrere Freiheitsgrade der Bewegung zur Positionierung des zu reinigenden sensiblen Bauteils relativ zu der Haube auf. Hierdurch kann insbesondere eine gezielte Ausrichtung des zu reinigenden Bauteils relativ zu dem Reinigungskopf ermöglicht werden. Hierdurch kann ein vorteilhaftes Reinigungsergebnis erzielt werden. Ferner kann hierdurch eine vorteilhaft komfortable Reinigung ermöglicht werden.

Ferner geht die Erfindung aus von einem Reinigungssystem mit der portablen Reinigungsvorrichtung und mit einem Anschlussmodul, welches zumindest zu einer Bereitstellung des Reinigungsfluids und/oder des Trocknungsfluids für die portable Reinigungsvorrichtung vorgesehen ist. Hierdurch kann insbesondere ein vorteilhaft kompaktes Reinigungssystem bereitgestellt werden. Vorzugsweise kann das Anschlussmodul mit verschiedenen portablen, insbesondere erfindungsgemäßen, Reinigungsvorrichtungen genutzt werden und/oder die portable Reinigungsvorrichtung kann mit verschiedenen Anschlussmodulen genutzt werden. Auf diese Weise kann insbesondere ein vorteilhaft variables Reinigungssystem bereitgestellt werden.

Es wird weiter vorgeschlagen, dass das Anschlussmodul zumindest einen ersten Tank zu einer Aufnahme des Reinigungsfluids, zumindest einen zweiten Tank zu einer Aufnahme des Trocknungsfluids und zumindest einen dritten Tank zu einer Aufnahme eines abgesaugten Reinigungsfluids aufweist. Dadurch kann insbesondere eine Aufnahme der Fluide in dem Anschlussmodul, und damit von der portablen Reinigungsvorrichtung entkoppelt, ermöglicht werden. Hierdurch kann insbesondere ein Gewicht der portablen Reinigungsvorrichtung gering gehalten werden.

Es wird ferner vorgeschlagen, dass das Reinigungssystem einen mit dem Anschlussmodul koppelbaren Kompressor aufweist, welcher dazu vorgesehen ist, das Reinigungsfluid des ersten Tanks unter Druck zu setzen und in dem dritten Tank einen Unterdruck zu erzeugen. Vorzugsweise dient der Kompressor insbesondere zu einem Ausbringen und zu einem Absaugen des Reinigungsfluids. Bevorzugt wird das Trocknungsfluid, insbesondere das Trocknungsgas, aus dem zweiten Tank mittels seines Eigengasdrucks ausgebracht. Das Trockungsfluid befindet sich insbesondere unter Druck in dem zweiten Tank. Dadurch kann insbesondere ein vorteilhaftes Reinigungssystem bereitgestellt werden. Der Kompressor kann vorzugsweise von einem bestehenden Komrpressor und/oder Druckluftsystem gebildet sein. Hierdurch kann insbesondere ein vorteilhaft modulares Reinigungssystem bereitgestellt werden.

### Zeichnungen

Weitere Vorteile ergeben sich aus der folgenden Zeichnungsbeschreibung. In den Zeichnungen sind zwei Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine erfindungsgemäße portable Reinigungsvorrichtung mit einem Reinigungskopf und einem Griffteil in einer schematischen Darstellung,
- Fig. 2: eine Vorderansicht des Reinigungskopfs der erfindungsgemäßen portablen Reinigungsvorrichtung, mit einer ersten Aufbringeinheit, mit einer zweiten Aufbringeinheit und mit einer Absaugeinheit in einer schematischen Darstellung und
- Fig. 3: eine alternative erfindungsgemäße portable Reinigungsvorrichtung mit einem Reinigungskopf, mit einem den Reinigungskopf führenden Schienensystem und mit einer den Reinigungskopf umschließenden Haube in einer schematischen Darstellung.

### Beschreibung der Ausführungsbeispiele

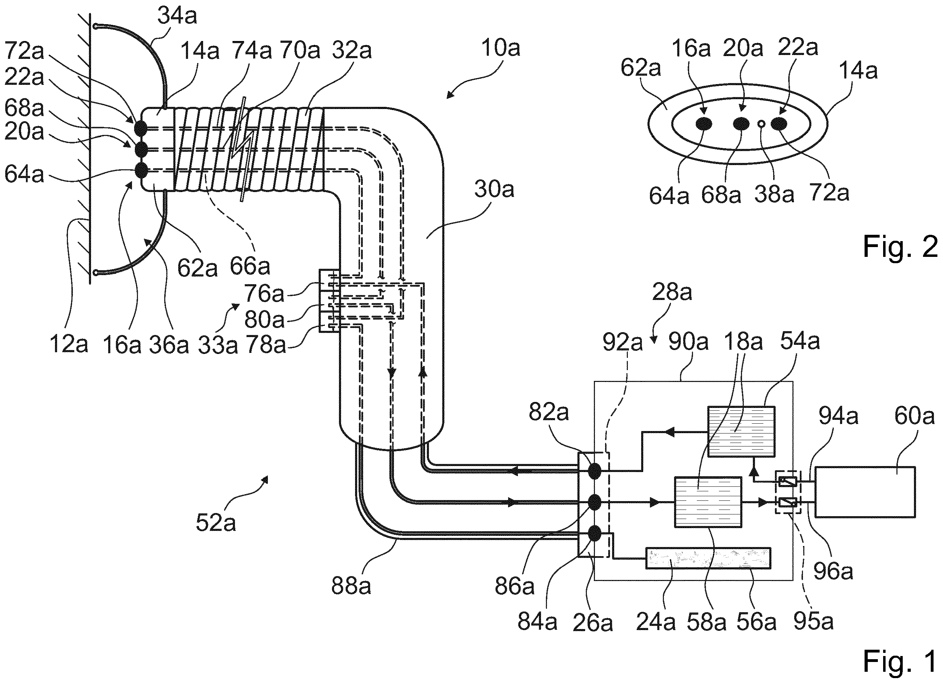

Die Figuren 1 und 2 zeigen ein Reinigungssystem 52a mit einer portablen Reinigungsvorrichtung 10a, mit einem Anschlussmodul 28a und mit einem mit dem Anschlussmodul 28a koppelbaren Kompressor 60a. Die portable Reinigungsvorrichtung 10a ist zur Flüssigreinigung sensibler Bauteile 12a vorgesehen. Die portable Reinigungsvorrichtung 10a ist zur Flüssigreinigung sensibler Luft- und Raumfahrtbauteile vorgesehen. Das schematisch dargestellte sensible Bauteil 12a ist beispielhaft von einem sensiblen Raumfahrtbauteil gebildet. Sensible Raumfahrtbauteile sind dabei beispielsweise Halterungen für optische Einrichtungen, Schrauben, Bolzen, Panel-Oberflächen (zur Entfernung von partikularen oder molekularen Kontaminanten oder zur Vorbereitung von Klebungen), Gehäuse von Subsystemen, wie z.B. On-board Computer, Massenspeicher, PCDU (Power Control Distribution Unit), etc. Die portable Reinigungsvorrichtung 10a ist von einem Bediener transportmaschinenlos transportierbar. Die portable Reinigungsvorrichtung 10a wird während eines Betriebs von einem Bediener manuell geführt und gehalten.

Die portable Reinigungsvorrichtung 10a weist einen Reinigungskopf 14a auf. Der Reinigungskopf 14a ist zu einer direkten Reinigung des sensiblen Bauteils 12a vorgesehen. Der Reinigungskopf 14a wird dazu insbesondere während einer Reinigung in die Nähe des zu reinigenden Bauteils 12a gebracht. Der Reinigungskopf 14a weist ein Gehäuse 62a auf. Ferner weist der Reinigungskopf 14a eine erste Aufbringeinheit 16a zu einem Aufbringen eines Reinigungsfluids 18a auf. Die erste Aufbringeinheit 16a umfasst dazu eine Düse 64a und einen mit der Düse 64a gekoppelten Führungskanal 66a. Die Aufbringeinheit 16a ist im Wesentlichen in dem Gehäuse 62a des Reinigungskopfs 14a angeordnet, wobei die Düse 64a aus dem Gehäuse 62a ragt. Die Düse 64a dient zu einem gezielten und definierten Ausbringen des Reinigungsfluids 18a. Der Führungskanal 66a dient zu einer Leitung des Reinigungsfluids 18a von einem Speicher zu der Düse 64a. Das Reinigungsfluid 18a ist von einer Reinigungsflüssigkeit gebildet. Das Reinigungsfluid 18a ist beispielhaft von Isopropanol gebildet. Ferner weist der Reinigungskopf 14a der portablen Reinigungsvorrichtung 10a eine Absaugeinheit 20a zu einem Absaugen des Reinigungsfluids 18a auf. Die Absaugeinheit 20a umfasst eine Absaugdüse 68a und eine mit der Absaugdüse 68a gekoppelten Absaugkanal 70a. Die Absaugeinheit 20a ist im Wesentlichen in dem Gehäuse 62a des Reinigungskopfs 14a angeordnet, wobei die Absaugdüse 68a aus dem Gehäuse 62a ragt. Die Absaugdüse 68a der Absaugeinheit 20a ist neben der Düse 64a der Aufbringeinheit 16a angeordnet und dient zu einem gezielten Absaugen des Reinigungsfluids 18a (Figuren 1, 2).

Des Weiteren weist der Reinigungskopf 14a eine weitere Aufbringeinheit 22a zum Aufbringen eines Trocknungsfluids 24a auf, zu einer Trocknung des sensiblen Bauteils 12a. Alternativ wäre auch denkbar, dass der Reinigungskopf 14a lediglich eine einzige Aufbringeinheit 16a aufweist, die zu einem Aufbringen des Reinigungsfluids 18a und des Trocknungsfluids 24a vorgesehen ist. Die weitere Aufbringeinheit 22a umfasst dazu eine Düse 72a und einen mit der Düse 72a gekoppelten Führungskanal 74a. Die weitere Aufbringeinheit 22a ist im Wesentlichen in dem Gehäuse 62a des Reinigungskopfs 14a angeordnet, wobei die Düse 72a aus dem Gehäuse 62a ragt. Die Düse 72a dient zu einem gezielten und definierten Ausbringen des Trocknungsfluids 24a. Die Düse 72a der weiteren Aufbringeinheit 22a ist neben der Absaugdüse 68a der Absaugeinheit 20a angeordnet. Grundsätzlich wäre jedoch auch eine andere, einem Fachmann als sinnvoll erscheinende Anordnung der Düsen 64a, 72a und der Absaugdüse 68a denkbar. Die Düsen 64a, 72a und die Absaugdüse 68a weisen jedoch insbesondere eine gleiche Ausrichtung auf. Der Führungskanal 74a der weiteren Aufbringeinheit 22a dient zu einer Leitung des Trocknungsfluids 24a von einem Speicher zu der Düse 72a. Das Trocknungsfluid 24a ist von einem Trocknungsgas gebildet. Das Trocknungsfluid 24a ist beispielhaft von Stickstoff (N₂) gebildet (Figuren 1, 2).

Ferner weist der Reinigungskopf 14a eine flexible, die Aufbringeinheiten 16a, 22a und die Absaugeinheit 20a zumindest teilweise umgreifende Schirmeinheit 34a auf. Die Schirmeinheit 34a umgreift die Düsen 64a, 72a der Aufbringeinheiten 16a, 22a und die Absaugdüse 68a der Absaugeinheit 20a. Die Schirmeinheit 34a ist zu einer Abschirmung eines Reinigungsbereichs 36a vorgesehen. Ferner dient die Schirmeinheit 34b dazu, andere Bauteile und/oder Bereiche des Bauteils 12a, beispielsweise bereits gereinigte Bauteile und/oder Bereiche des Bauteils 12a, zu schützen. Die Schirmeinheit 34a definiert den Reinigungsbereich 36a. Die portable Reinigungsvorrichtung 10a ist insbesondere zu einer Reinigung von Bauteilen 12a vorgesehen, die größer sind als der Reinigungsbereich 36a. Die Schirmeinheit 34a begrenzt daher einen definierten Reinigungsbereich 36a auf dem Bauteil 12, welcher jeweils aktuell gereinigt wird. Ein Bediener erhält dadurch eine Rückmeldung über eine Größe des aktuell gereinigten Reinigungsbereich 36a des Bauteils 12a. Zudem wird mittels der Schirmeinheit 34a ein räumlicher Bereich zu einer Reinigung abgeschirmt. Die Schirmeinheit 34a ist dazu vorgesehen, ein Verspritzen des Reinigungsfluids 18a aus dem Reinigungsbereich 36a heraus zu vermeiden. Ferner ist die Schirmeinheit 34a dazu vorgesehen, eine Saugwirkung der Absaugeinheit 20a zu verbessern. Die Schirmeinheit 34a ist glockenförmig ausgebildet, wobei ein von der Schirmeinheit 34a umgriffener und zumindest teilweise begrenzter Raum den Reinigungsbereich 36a bildet. Die Schirmeinheit 34a besteht aus einem elastischen Material, insbesondere aus einem elastischen Kunststoff. Ferner besteht die Schirmeinheit 34a aus einem transparenten Material, insbesondere einem elastischen und transparenten Kunststoff. Es wäre jedoch auch ein anderes, einem Fachmann als sinnvoll erscheinendes Material denkbar. Die Aufbringeinheiten 16a, 22a und die Absaugeinheit 20a ragen durch eine den Reinigungskopf 14a dicht umgreifende erste Öffnung in der Schirmeinheit 34a in den Reinigungsbereich 36a. Zudem weist die Schirmeinheit 34a eine der ersten Öffnung gegenüberliegende zweite Öffnung auf, welche zu einer Angrenzung an das sensible Bauteil 12a vorgesehen ist. Die Schirmeinheit 34a ist insbesondere abnehmbar ausgeführt.

Der Reinigungskopf 14a umfasst ferner ein Beleuchtungselement 38a. Das Beleuchtungselement 38a ist von einem LED-Element, insbesondere von einer einzelnen Leuchtdiode oder einem LED-Array, gebildet. Das Beleuchtungselement 38a ist in das Gehäuse 62a des Reinigungskopfes 14a integriert und ist dazu vorgesehen, in den Reinigungsbereich 36a zu strahlen. Das Beleuchtungselement 38a ist beispielhaft zwischen der Düse 64a der Aufbringeinheit 16a und der Absaugdüse 68a der Absaugeinheit 20a angeordnet. Es wäre jedoch auch eine andere, einem Fachmann als sinnvoll erscheinende Anordnung des Beleuchtungselements 38a denkbar. Das Beleuchtungselement 38a ist zu einer optischen Ausleuchtung des Reinigungsbereichs 36a vorgesehen. Alternativ oder zusätzlich kann das Beleuchtungselement 38a jedoch auch von einem UV-Beleuchtungselement 38a gebildet sein, das zu einer biologischen Dekontamination des Bauteils 12a, insbesondere zur Entfernung von Mikroben und Sporen, etc., vorgesehen ist (Figur 2).

Ferner weist die portable Reinigungsvorrichtung 10a einen Griffteil 30a auf, an welchem der Reinigungskopf 14a angeordnet ist. Der Griffteil 30a ist von einem Gehäuseteil der Reinigungsvorrichtung 10a gebildet, der als Griff für einen Bediener dient. Der Griffteil 30a ist beispielhaft von einem Pistolengriff gebildet. Grundsätzlich wäre jedoch auch eine andere, einem Fachmann als sinnvoll erscheinende Ausbildung des Griffteils 30a denkbar. Ferner wäre denkbar, dass der Griffteil 30a zu einer Verbesserung einer Ergonomie und/oder eines Bedienkomforts eine Beschichtung aufweist. Der Griffteil 30a ist zu einer manuellen Führung des Reinigungskopfs 14a vorgesehen. Der Reinigungskopf 14a ist dazu mit dem Griffteil 30a gekoppelt. Zudem weist die portable Reinigungsvorrichtung 10a eine zwischen dem Griffteil 30a und dem Reinigungskopf 14a angeordnete Verstelleinheit 32a auf. Mittels der Verstelleinheit 32a ist eine räumliche Ausrichtung des Reinigungskopfs 14a relativ zu dem Griffteil 30a einstellbar. Die Verstelleinheit 32a ist von einem den Griffteil 30a und den Reinigungskopf 14a verbindenden Metallschlauch gebildet. Der Führungskanal 66a der ersten Aufbringeinheit 16a, der Führungskanal 74a der weiteren Aufbringeinheit 22a und der Absaugkanal 70a der Absaugeinheit 20a sind durch die Verstelleinheit 32a hindurch zu dem Griffteil 30a geführt. Die Verstelleinheit 32a kann dabei, insbesondere abhängig von einer Ausgestaltung der portablen Reinigungsvorrichtung 10a, verschiedene Längen aufweisen, wobei insbesondere eine Länge zwischen 10 cm und 50 cm vorteilhaft ist.

Die portable Reinigungsvorrichtung 10a weist ferner eine an dem Griffteil 30a angeordnete Bedieneinheit 33a auf, welche zu einer Betätigung der Aufbringeinheiten 16a, 22a und der Absaugeinheit 20a vorgesehen ist. Die Bedieneinheit 33a weist ein erstes Betätigungselement 76a auf, welches zu einer Versorgung der Aufbringeinheit 16a mit Reinigungsfluid 18a vorgesehen ist. Das Betätigungselement 76a umfasst dazu insbesondere ein Ventil, welches den Führungskanal 66a der ersten Aufbringeinheit 16a in einem betätigten Zustand des Betätigungselements 76a mit einem Tank 54a für das Reinigungsfluid 18a koppelt. Ferner weist die Bedieneinheit 33a ein zweites Betätigungselement 78a auf, welches zu einer Versorgung der weiteren Aufbringeinheit 22a mit Trocknungsfluid 24a vorgesehen ist. Das Betätigungselement 78a umfasst dazu insbesondere ein Ventil, welches den Führungskanal 74a der weiteren Aufbringeinheit 22a in einem betätigten Zustand des Betätigungselements 78a mit einem Tank 56a für das Trocknungsfluid 24a koppelt. Des Weiteren weist die Bedieneinheit 33a ein drittes Betätigungselement 80a auf, welches zu einer Kopplung der Absaugeinheit 20a mit einer Absaugung vorgesehen ist. Das Betätigungselement 80a umfasst dazu insbesondere ein Ventil, welches den Absaugkanal 70a der Absaugeinheit 20a in einem betätigten Zustand des Betätigungselements 80a mit einer Absaugung koppelt. Es wäre auch denkbar, dass die Bedieneinheit 33a lediglich ein Betätigungselement aufweist, welches zu einer Betätigung der Aufbringeinheiten 16a, 22a und der Absaugeinheit 20a vorgesehen ist. Die Betätigungselemente 76a, 78a, 80a der Bedieneinheit 33a sind jeweils von einem Druckknopf gebildet. Es wäre jedoch auch eine andere, einem Fachmann als sinnvoll erscheinende Ausgestaltung der Betätigungselemente 76a, 78a, 80a denkbar.

Des Weiteren weist die portable Reinigungsvorrichtung 10a eine Kopplungseinheit 26a auf, die zu einer lösbaren Kopplung der Reinigungsvorrichtung 10a mit dem Anschlussmodul 28a vorgesehen ist. Die Kopplungseinheit 26a stellt Anschlüsse 82a, 84a, 86a zur Bereitstellung und zur Abfuhr des Reinigungsfluids 18a und zur Bereitstellung des Trocknungsfluids 24a bereit. Die Kopplungseinheit 26a weist einen Anschluss 82a zu einer Bereitstellung des Reinigungsfluids 18a und einen Anschluss 84a zu einer Bereitstellung des Trocknungsfluids 24a auf, wobei das Reinigungsfluid 18a und das Trocknungsfluid 24a von dem Anschlussmodul 28a bereitgestellt werden. Ferner weist die Kopplungseinheit 26a einen weiteren Anschluss 86a zu einer Abfuhr des abgesaugten Reinigungsfluids 18a auf. Die Anschlüsse 82a, 84a, 86a sind jeweils in Form einer Steckverbindung realisiert und sind in einem gemeinsamen Steckverbinder gekoppelt. Die Kopplungseinheit 26a bildet den Steckverbinder aus und ist dazu vorgesehen, direkt in das Anschlussmodul 28a eingesteckt zu werden, wobei gleichzeitig alle Anschlüsse 82a, 84a, 86a mit dem Anschlussmodul 28a gekoppelt werden. Die Kopplungseinheit 26a ist fest mit dem Reinigungskopf 14a gekoppelt und lösbar mit dem Anschlussmodul 28a koppelbar. Die Kopplungseinheit 26a ist über eine Leitung 88a, welche drei Teilkanäle zu einer Führung des Reinigungsfluids 18a, des Trocknungsfluids 24a und des abgesaugten Reinigungsfluids 18a umfasst, mit dem Griffteil 30a und in dem Griffteil 30a mit der Bedieneinheit 33a verbunden. Über die Leitung 88a ist der erste Anschluss 82a mit dem ersten Betätigungselement 76a der Bedieneinheit 33a, der zweite Anschluss 84a mit dem zweiten Betätigungselement 78a der Bedieneinheit 33a und der dritte Anschluss 86a mit dem dritten Betätigungselement 80a der Bedieneinheit 33a gekoppelt.

Das Anschlussmodul 28a ist von einem separaten Modul gebildet. Das Anschlussmodul 28a kann daher getrennt von der portablen Reinigungsvorrichtung 10a transportiert werden. Das Anschlussmodul 28a ist zu einer Bereitstellung des Reinigungsfluids 18a und des Trocknungsfluids 24a für die portable Reinigungsvorrichtung 10a vorgesehen. Ferner ist das Anschlussmodul 28a zu einer Aufnahme des abgesaugten, verunreinigten Reinigungsfluids 18a vorgesehen. Das Anschlussmodul 28a weist einen ersten Tank 54a zu einer Aufnahme des Reinigungsfluids 18a, den zweiten Tank 56a zu einer Aufnahme des Trocknungsfluids 24a und einen dritten Tank 58a zu einer Aufnahme des abgesaugten Reinigungsfluids 18a auf. Der zweite Tank 56a ist von einer insbesondere austauschbaren Druckflasche zu einer Aufnahme eines unter Druck stehenden Gases gebildet. Der zweite Tank 56a verfügt zudem über einen nicht weiter dargestellten Druckregler. Ferner weist das Anschlussmodul 28a ein Gehäuse 90a auf, in welchem die Tanks 54a, 56a, 58a aufgenommen sind. Zudem weist das Anschlussmodul 28a eine mit den Tanks 54a, 56a, 58a gekoppelte Kopplungseinheit 92a auf, welche zu einer direkten Kopplung mit der Kopplungseinheit 26a der portablen Reinigungsvorrichtung 10a vorgesehen ist.

Zudem wird für das Reinigungssystem 52a der mit dem Anschlussmodul 28a koppelbare Kompressor 60a benötigt. Der Kompressor 60a kann dabei insbesondere von einem beliebigen, insbesondere bereits vorhandenen Kompressor gebildet sein. Für einen Betrieb ist das Anschlussmodul 28a über eine erste Leitung 94a und eine Einstelleinheit 95a des Anschlussmoduls 28a mit einem Druckausgang des Kompressors 60a und über eine zweite Leitung 96a und die Einstelleinheit 95a des Anschlussmoduls 28a mit einem Ansaugeingang des Kompressors 60a gekoppelt. Die erste Leitung 94a ist mit dem ersten Tank 54a verbunden, während die zweite Leitung 96a mit dem dritten Tank 58a verbunden ist. Der Kompressor 60a ist dazu vorgesehen, das Reinigungsfluid 18a des ersten Tanks 54a unter Druck zu setzen und in dem dritten Tank 58a einen Unterdruck zu erzeugen. Mittels der Einstelleinheit 95a kann ein Druck in dem ersten Tank 54a eingestellt und geregelt werden. Ferner kann mittels der Einstelleinheit 95a zudem ein Unterdruck in dem dritten Tank 58a eingestellt und geregelt werden.

Bei einer Betätigung des ersten Betätigungselements 76a der Bedieneinheit 33a wird das unter Druck stehende Reinigungsfluid 18a aus dem ersten Tank 54a über die Leitung 88a in den Führungskanal 66a der ersten Aufbringeinheit 16a gefördert und wird von dort über die Düse 64a verteilt. Bei einer Betätigung des zweiten Betätigungselements 78a der Bedieneinheit 33a wird das unter Druck stehende Trocknungsfluid 24a aus dem zweiten Tank 56a über die Leitung 88a in den Führungskanal 74a der weiteren Aufbringeinheit 22a gefördert und wird von dort über die Düse 72a verteilt. Bei einer Betätigung des dritten Betätigungselements 80a der Bedieneinheit 33a wird die Absaugdüse 68a über den Absaugkanal 70a und die Leitung 88a mit dem dritten Tank 58a verbunden. Durch den Unterdruck in dem dritten Tank 58a entsteht eine Sogwirkung, sodass über die Absaugdüse 68a Reinigungsfluid 18a aus dem Reinigungsbereich 36a angesaugt wird.

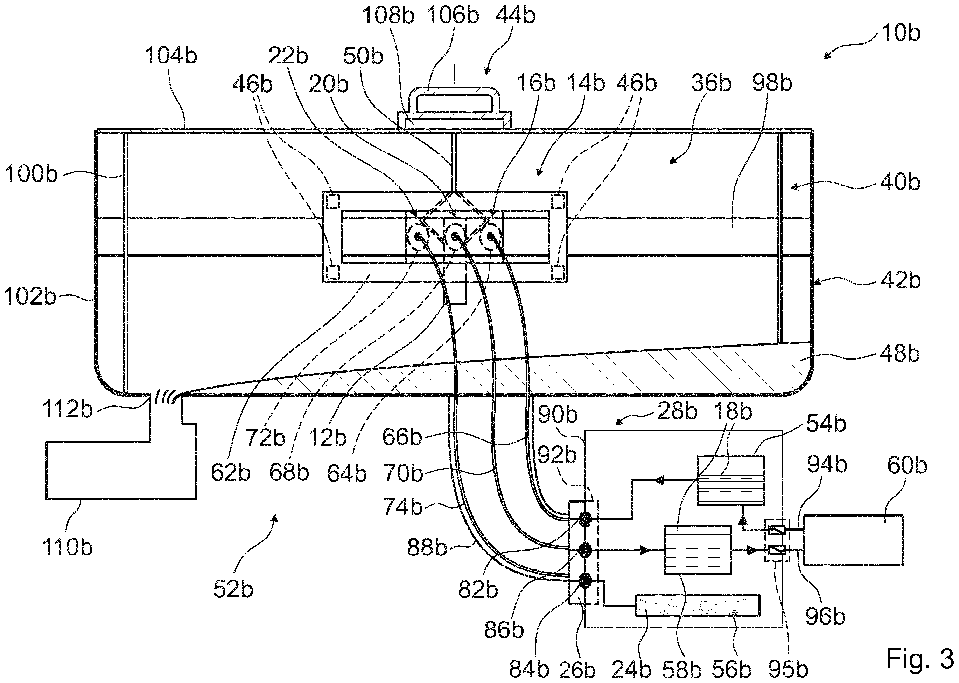

In der Figur 3 ist ein weiteres Ausführungsbeispiel der Erfindung gezeigt. Die nachfolgende Beschreibung beschränkt sich im Wesentlichen auf die Unterschiede zwischen den Ausführungsbeispielen, wobei bezüglich gleich bleibender Bauteile, Merkmale und Funktionen auf die Beschreibung des Ausführungsbeispiels der Figuren 1 und 2 verwiesen werden kann. Zur Unterscheidung der Ausführungsbeispiele ist der Buchstabe a in den Bezugszeichen des Ausführungsbeispiels in den Figuren 1 und 2 durch den Buchstaben b in den Bezugszeichen des Ausführungsbeispiels der Figur 3 ersetzt. Bezüglich gleich bezeichneter Bauteile, insbesondere in Bezug auf Bauteile mit gleichen Bezugszeichen, kann grundsätzlich auch auf die Zeichnungen und/oder die Beschreibung des Ausführungsbeispiels der Figuren 1 und 2 verwiesen werden.

Die Figur 3 zeigt ein Reinigungssystem 52b mit einer portablen Reinigungsvorrichtung 10b (diese in Seitenansicht dargestellt), mit einem Anschlussmodul 28b und mit einem mit dem Anschlussmodul 28b koppelbaren Kompressor 60b. Die portable Reinigungsvorrichtung 10b ist zur Flüssigreinigung sensibler Bauteile 12b vorgesehen. Die portable Reinigungsvorrichtung 10b ist von einem Bediener transportmaschinenlos transportierbar. Die portable Reinigungsvorrichtung 10b ist während eines Betriebs feststehend, wobei ein Reinigungskopf 14b der portablen Reinigungsvorrichtung 10b von einem Bediener manuell geführt und bewegt wird.

Die portable Reinigungsvorrichtung 10b weist den Reinigungskopf 14b auf. Ferner weist die portable Reinigungsvorrichtung 10b ein eine Bewegung des Reinigungskopfs 14b in mindestens zwei Dimensionen ermöglichendes Schienensystem 40b auf. Das Schienensystem 40b ermöglicht eine Bewegung des Reinigungskopfs 14b in drei Dimensionen. Grundsätzlich wäre jedoch auch denkbar, dass das Schienensystem 40b lediglich eine Bewegung des Reinigungskopfs 14b in zwei Dimensionen ermöglicht. Das Schienensystem 40b weist eine erste horizontale Führungsschiene 98b auf. Der Reinigungskopf 14b ist/sind direkt an der ersten Führungsschiene 98b horizontal geführt. Ferner weist das Schienensystem 40b eine nicht weiter sichtbare, zweite horizontale Führungsschiene auf. Die erste Führungsschiene 98b, und damit indirekt auch der Reinigungskopf 14b, ist an der zweiten Führungsschiene horizontal, senkrecht zu der ersten Führungsschiene 98b geführt. Des Weiteren weist das Schienensystem 40b eine dritte, vertikale Führungsschiene 100b auf. Die zweite Führungsschiene, und über diese auch die erste Führungsschiene 98b und der Reinigungskopf 14b, ist/sind an der dritten Führungsschiene 100b vertikal geführt. Der Reinigungskopf 14b kann dadurch in einem Bewegungsradius der Führungsschienen 98b, 100b in drei Dimensionen mittels des Schienensystems 40b geführt werden.

Ferner weist die portable Reinigungsvorrichtung 10b eine, den Reinigungskopf 14b und das zu reinigende Bauteil 12b von mehreren Seiten umschließende Haube 42b auf. Die Haube 42b besteht bespielhaft aus einem quaderförmigen Behälter 102b mit einem Deckel 104b. Die Haube 42b nimmt den Reinigungskopf 14b und das Schienensystem 40b auf. Das Schienensystem 40b ist vollständig innerhalb der Haube 42b angeordnet. Die Haube 42b dient zu einem Abschirmen eines Reinigungsbereichs 36b. Gleichzeitig dient die Haube 42b insbesondere zu einem Transport der portablen Reinigungsvorrichtung 10b. Die Haube 42b kann dazu insbesondere nicht weiter dargestellte Griffe oder dergleichen aufweisen. Ferner besteht die Haube 42b zumindest teilweise aus einem transparenten Material. Die Haube 42b besteht beispielhaft aus einem transparenten Kunststoff. Die Haube 42b besteht insbesondere aus Acrylglas. Es wäre jedoch auch ein anderes, einem Fachmann als sinnvoll erscheinendes Material denkbar.

Ferner weist die portable Reinigungsvorrichtung 10b eine unterhalb des Reinigungskopfs 14b angeordnete Aufnahmewanne 48b auf, die zur Aufnahme von nicht abgesaugtem Reinigungsfluid 18b vorgesehen ist. Die Aufnahmewanne 48b bildet einen Boden zu der Haube 42b aus. Die Aufnahmewanne 48b bildet einen Teil des Behälters 102b aus. Grundsätzlich wäre jedoch auch denkbar, dass die Aufnahmewanne 48b separat ausgeführt ist. Ferner weist die Aufnahmewanne 48b einen mit einem Auffangbehälter 110b gekoppelten Abfluss 112b auf. Der Auffangbehälter 110b ist direkt mit dem Abfluss 112b gekoppelt und insbesondere abnehmbar ausgeführt. Der Auffangbehälter 110b dient zu einer Aufnahme verschmutzten Reinigungsfluids 18b. Hierdurch kann eine einfache Leerung des Auffangbehälters 110b ermöglicht werden. Ferner weist die Aufnahmewanne 48b bei einem ebenen Stand der portablen Reinigungsvorrichtung 10b ein Gefälle in Richtung des Abflusses 112b auf. Das Gefälle beträgt beispielhaft annähernd 2°.

Des Weiteren weist die portable Reinigungsvorrichtung 10b ein auf der Haube 42b bewegliches, handgeführtes magnetisches Führungselement 44b auf, welches zu einer indirekten Bewegung des Reinigungskopfs 14b vorgesehen ist. Das Führungselement 44b ist zu einer Führung auf der äußeren Oberfläche der Haube 42b vorgesehen. In der Darstellung der Fig. 3 befindet sich das Führungselement auf dem Deckel 104b der Haube 42b. Das Führungselement 44b kann dabei sowohl frei beweglich als auch definiert geführt ausgeführt sein. Das Führungselement 44b ist beispielhaft frei beweglich ausgeführt und kann von einem Bediener frei auf dem Deckel 104b oder dem Behälter 102b der Haube 42b bewegt werden. Das Führungselement 44b ist von einem Magnetgriff gebildet. Das Führungselement 44b weist einen Grundkörper 106b auf, welcher einen Griffbereich des Führungselements 44b ausbildet. Der Grundkörper 106b ist beispielhaft bügelförmig ausgebildet. Ferner umfasst das Führungselement 44b ein fest mit dem Grundkörper 106b verbundenes Magnetelement 108b auf. Das Magnetelement 108b ist an einer Unterseite des Grundkörpers 106b angeordnet. Das Magnetelement 108b wird in einem Betrieb mittels des Grundkörpers 106b direkt über den Deckel 104b oder dem Behälter der der Haube 42b geführt. Das Magnetelement 108b ist beispielhaft von einem Permanentmagneten gebildet. Das Magnetelement 108b ist von einem Neodymmagneten gebildet. Es wäre jedoch auch eine andere, einem Fachmann als sinnvoll erscheinende Ausbildung des Magnetelements 108b denkbar. Zudem weist der Reinigungskopf 14b zumindest einen Permanentmagneten 46b auf. Der Permanentmagnet 46b ist von einem Neodymmagneten gebildet. Der Permanentmagnet 46b des Reinigungskopfs 14b ist dazu vorgesehen, in einem Betrieb mit dem Magnetelement 108b zusammenzuwirken und eine mit dem Führungselement 44b synchrone Bewegung des Reinigungskopfes 14b zu bewirken. Der Reinigungskopf 14b kann so indirekt mittels des Führungselements 44b bewegt werden. Der Reinigungskopf 14b kann so von außerhalb der Haube 42b mittels des Führungselements 44b von Hand bewegt werden.

Zudem weist die portable Reinigungsvorrichtung 10b eine Halteeinheit 50b auf, welche zu einer im Wesentlichen freien Aufnahme des zu reinigenden sensiblen Bauteils 12b vorgesehen ist. Die Halteeinheit 50b ist an der Haube 42b angeordnet. Die Halteeinheit 50b ist beispielhaft an dem Deckel 104b der Haube 42b angeordnet. Es wäre jedoch auch denkbar, dass die Halteeinheit 50b an einer oder mehreren der Innenwände des Behälters 102b der Haube 42b oder an der Aufnahmewanne 48b verankert ist. Die Halteeinheit 50b ist zu einem Einspannen des Bauteils 12b vorgesehen. Das Bauteil 12b kann dabei insbesondere über zwei einander gegenüberliegende Seitenkanten in der Halteeinheit 50b eingespannt werden. Die Halteeinheit 50b ist dazu zangenartig ausgeführt. Es wäre jedoch auch eine andere, einem Fachmann als sinnvoll erscheinende Ausgestaltung der Halteeinheit 50b denkbar, wie beispielsweise mittels eines Saufnapfs. Die Halteeinheit 50b weist mehrere Freiheitsgrade einer Bewegung zur Positionierung des zu reinigenden sensiblen Bauteils 12b auf. Die Halteeinheit 50b ist dazu beweglich an der Haube 42b angeordnet. Die Halteeinheit 50b kann relativ zu der Haube 42b verdreht werden, insbesondere um eine vertikale Achse. Zudem wäre denkbar, dass mittels der Halteeinheit 50b eine Höhe des Bauteils 12b innerhalb der Haube 42b eingestellt werden kann. Ferner wäre denkbar, dass die Positionierung der Halteeinheit 50b innerhalb der Haube 42 von außerhalb der Haube 42b bewirkt werden kann. Vorzugsweise weist die Haube 42b dazu, insbesondere in dem Deckel 104b, eine Führungsnut auf, in welcher die Halteeinheit 50b geführt ist. Alternativ könnte eine Veränderung der Lage des zu reinigenden Bauteils mittels einer horizontalen Drehachse bewerkstelligt werden, die sich an gegenüberliegenden Innenwänden des Behälters 102b abstützt.

Ferner weist die portable Reinigungsvorrichtung 10b eine nicht weiter dargestellte Bedieneinheit auf, welche zu einer Betätigung von Aufbringeinheiten 16b, 22b und einer Absaugeinheit 20b vorgesehen ist. Die Bedieneinheit ist entsprechend der Bedieneinheit 33a des ersten Ausführungsbeispiels der Figuren 1 und 2 ausgeführt. Die Bedieneinheit kann beispielsweise an dem Führungselement 44b angeordnet sein. Alternativ kann die Bedieneinheit beispielsweise als ein Fußschalter ausgeführt sein.

Des Weiteren weist die portable Reinigungsvorrichtung 10b eine Kopplungseinheit 26b auf, die zu einer lösbaren Kopplung der Reinigungsvorrichtung 10b mit dem Anschlussmodul 28b vorgesehen ist. Das Anschlussmodul 28b ist von einem separaten Modul gebildet. Das Anschlussmodul 28b ist entsprechend dem Anschlussmodul 28a des ersten Ausführungsbeispiels der Figuren 1 und 2 ausgeführt. Das Anschlussmodul 28b kann daher insbesondere für beide portable Reinigungsvorrichtungen 10a, 10b beider Ausführungsbeispiele verwendet werden. Es kann daher insbesondere vorgesehen sein, dass ein Reinigungssystem 52b aus zwei portablen Reinigungsvorrichtungen 10a, 10b und lediglich einem Anschlussmodul 28b besteht, wobei jeweils abhängig von einer Anwendung eine andere der Reinigungsvorrichtungen 10a, 10b zusammen mit dem Anschlussmodul 28b verwendet wird.

Zudem wird für das Reinigungssystem 52b der mit dem Anschlussmodul 28b koppelbare Kompressor 60b benötigt.

## Patentansprüche

1. Portable Reinigungsvorrichtung zur Flüssigreinigung sensibler Bauteile (12b), insbesondere sensibler Luft- und/oder Raumfahrtbauteile, mit zumindest einem Reinigungskopf (14b), welche zumindest eine Aufbringeinheit (16b) zu einem Aufbringen eines Reinigungsfluids (18b) und zumindest eine Absaugeinheit (20b) zu einem Absaugen des Reinigungsfluids (18b) aufweist,
wobei
der Reinigungskopf (14b) zumindest eine Aufbringeinheit (22b) zum Aufbringen eines Trocknungsfluids (24b), insbesondere eines Trocknungsgases, zu einer Trocknung des sensiblen Bauteils (12b) aufweist, **gekennzeichnet durch** zumindest eine, den Reinigungskopf (14b) und das zu reinigende Bauteil (12b) von mehreren Seiten umschließende Haube (42b), welche zumindest teilweise aus einem transparenten Material besteht und durch zumindest ein auf der Haube (42b) bewegliches, handgeführtes magnetisches Führungselement (44b), welches zu einer indirekten Bewegung des Reinigungskopfs (14b) vorgesehen ist, wobei der Reinigungskopf (14b) einen Permanentmagneten (46b) aufweist.

2. Portable Reinigungsvorrichtung nach Anspruch 1,
**gekennzeichnet durch**
zumindest eine Kopplungseinheit (26b), die zu einer lösbaren Kopplung der Reinigungsvorrichtung (10b) mit einem Anschlussmodul (28b) vorgesehen ist, wobei die Kopplungseinheit (26b) zumindest Anschlüsse zur Bereitstellung und zur Abfuhr des Reinigungsfluids (18b) und des Trocknungsfluids (24b) aufweist.

3. Portable Reinigungsvorrichtung nach einem der vorhergehenden Ansprüche, **gekennzeichnet durch**
ein eine Bewegung des Reinigungskopfs (14b) in mindestens zwei Dimensionen ermöglichendes Schienensystem (40b).

4. Portable Reinigungsvorrichtung nach einem der vorhergehenden Ansprüche, **dadurch gekennzeichnet, dass**
unterhalb des Reinigungskopfs (14b) zumindest eine Aufnahmewanne (48b) zur Aufnahme von nicht abgesaugtem Reinigungsfluid (18b) und/oder Trocknungsfluid (24b) vorhanden ist.

5. Portable Reinigungsvorrichtung nach einem der vorhergehenden Ansprüche, **gekennzeichnet durch**
zumindest eine Halteeinheit (50b), welche zu einer im Wesentlichen freien Aufnahme des zu reinigenden sensiblen Bauteils (12b) vorgesehen ist.

6. Portable Reinigungsvorrichtung nach einem der vorhergehenden Ansprüche, **dadurch gekennzeichnet, dass**
die Halteeinheit (50b) mehrere Freiheitsgrade einer Bewegung zur Positionierung des zu reinigenden sensiblen Bauteils (12b) aufweist.

## Claims

1. Portable cleaning device for liquid cleaning of sensitive components (12b), in particular sensitive aviation and/or space travel components, with at least one cleaning head (14b) comprising at least one application unit (16b) for an application of a cleaning fluid (18b) and at least one aspiration unit (20b) for an aspiration of the cleaning fluid (18b),
wherein the cleaning head (14b) comprises at least one application unit (22b) for an application of a drying fluid (24b), in particular a drying gas, for drying the sensitive component (12b),
**characterised by**
at least one hood (42b) encompassing the cleaning head (14b) and the component (12b) that is to be cleaned from several sides, said hood (42b) being made at least partially of a transparent material, and by at least one manually guided magnetic guiding element (44b) which is movable on the hood (42b) and is configured for an indirect movement of the cleaning head (14b),
the cleaning head (14b) comprising a permanent magnet (46b).

2. Portable cleaning device according to claim 1,
**characterised by**
at least one coupling unit (26b) which is configured for a releasable coupling of the cleaning device (10b) with a connection module (28b),
the coupling unit (26b) comprising at least connections for supplying and for transporting away the cleaning fluid (18b) and the drying fluid (24b).

3. Portable cleaning device according to one of the preceding claims,
**characterised by**
a rail system (40b) permitting a movement of the cleaning head (14b) in at least two dimensions.

4. Portable cleaning device according to one of the preceding claims, **characterised in that**
below the cleaning head (14b) there is at last one receiving tub (48b) for receiving non-suctioned cleaning fluid (18b) and/or drying fluid (24b).

5. Portable cleaning device according to one of the preceding claims, **characterised by**
at least one holding unit (50b) which is configured for a substantially free receiving of the sensitive component (12b) that is to be cleaned.

6. Portable cleaning device according to one of the preceding claims, **characterised in that**
the holding unit (50b) has several degrees of freedom of a movement for positioning the sensitive component (12b) that is to be cleaned.

## Revendications

1. Dispositif de nettoyage portable pour le nettoyage liquide des composants sensibles (12b), en particulier des composants structurels sensibles d'aviation et/ou d'astronautique, avec au moins une tête de nettoyage (14b) comprenant au moins une unité d'application (16b) pour appliquer un fluide de nettoyage (18b) et au moins une unité d'aspiration (20b) pour l'aspiration du fluide de nettoyage (18b),
la tête de nettoyage (14b) comprenant au moins une unité d'application (22b) pour appliquer un fluide de séchage (24b), en particulier un gas de séchage, pour le séchage du composant sensible (12b),
**caractérisé par**
au moins un capot (42b) entourant de plusieurs côtés la tête de nettoyage (14b) et le composant (12b) qui est à nettoyer, ledit capot (42b) étant réalisé au moins partiellement d'un matériau transparent, et par au moins un élément de guidage magnétique (44b) qui est guidé manuellement et est mouvable sur le capot (42b) et qui est prévu pour un mouvement indirect de la tête de nettoyage (14b),
la tête de nettoyage (14b) comportant un aimant permanent (46b).

2. Dispositif de nettoyage portable selon la revendication 1,
**caractérisé par**
au moins une unité d'accouplement (26b) qui est pourvue d'un module de raccordement (28b) pour un accouplement relâchable du dispositif de nettoyage (10b), l'unité d'accouplement (26b) comprenant au moins des raccordements pour alimenter et pour amener le fluide de nettoyage (18b) et le fluide de séchage (24b).

3. Dispositif de nettoyage portable selon l'une des revendications précédentes, **caractérisé par**
un système de rails (40b) permettant un mouvement de la tête de nettoyage (14b) dans au moins deux dimensions.

4. Dispositif de nettoyage portable selon l'une des revendications précédentes, **caractérisé en ce qu'**
au-dessous de la tête de nettoyage (14b) il y a au moins une cuve de logement (48b) pour recevoir de fluide (18b) non-aspiré et/ou de fluide de séchage (24b) non-aspiré

5. Dispositif de nettoyage portable selon l'une des revendications précédentes, **caractérisé par**
au moins une unité de retenue (50b) qui est prévue pour un sensiblement libre logement du composant sensible (12b) qui est à nettoyer.

6. Dispositif de nettoyage portable selon l'une des revendications précédentes, **caractérisé en ce que**
l'unité de retenue (50b) présente plusieurs degrés de liberté d'un mouvement pour positionner le composant sensible (12b) qui est à nettoyer.
